# EUROPEAN PATENT SPECIFICATION

(11) **EP 1 820 023 B1**
(45) Date of publication and mention of the grant of the patent: **13.05.2009**
(21) Application number: 05815640.7
(22) Date of filing: 14.11.2005
(51) Int. Cl.: G01N 33/569, A61K 35/00, C12N 5/00

(54) **STEM CELLS, METHOD FOR THEIR PURIFICATION, IDENTIFICATION, AND USE**
STAMMZELLEN, VERFAHREN ZU DEREN REINIGUNG, IDENTIFIZIERUNG UND VERWENDUNG
CELLULES SOUCHES, PROCEDE DE PURIFICATION, D'IDENTIFICATION ET D'UTILISATION DE CELLES-CI

(30) Priority: 15.11.2004 IT FI20040238
(43) Date of publication of application: 22.08.2007
(73) Proprietor: Azienda Ospedaliero-Universitaria Careggi, 50139 Firenze (IT)
(72) Inventor: ROMAGNANI, Paola, I-50139 Firenze (IT); ANNUNZIATO, Francesco, I-50143 Firenze (IT); MAGGI, Enrico, 50139 Firenze (IT); ROMAGNANI, Sergio, I-50139 Firenze (IT)
(74) Representative: Gervasi, Gemma
(86) International application number: PCT/EP2005/055950
(87) International publication number: WO 2006/051112

(56) References cited:
- HAMDI H K ET AL: "A genetic variant of ACE increases cell survival: a new paradigm for biology and disease" BIOCHEMICAL AND BIOPHYSICAL RESEARCH COMMUNICATIONS, ACADEMIC PRESS INC. ORLANDO, FL, US, vol. 318, no. 1, 21 May 2004 (2004-05-21), pages 187-191, XP004504088 ISSN: 0006-291X
- REHMAN J ET AL: "PERIPHERAL BLOOD ENDOTHELIAL PROGENITOR CELLS ARE DERIVED FROM MONOCYTE/MACROPHAGES AND SECRETE ANGIOGENIC GROWTH FACTORS" CIRCULATION, AMERICAN HEART ASSOCIATION, DALLAS, TX, US, vol. 107, no. 8, 24 February 2003 (2003-02-24), pages 1164-1169, XP008022340 ISSN: 0009-7322
- ANNUNZIATO FRANCESCO ET AL: "Phenotype, localization, and mechanism of suppression of CD4(+)CD25(+) human thymocytes." THE JOURNAL OF EXPERIMENTAL MEDICINE. 5 AUG 2002, vol. 196, no. 3, 5 August 2002 (2002-08-05), pages 379-387, XP002370078 ISSN: 0022-1007
- SCHEFFOLD A ET AL: "High-sensitive immunofluorescence for detection of the pro- and anti-inflammatory cytokines gamma interferon and interleukin-10 on the surface of cytokine-secreting cells" NATURE MEDICINE, NATURE PUBLISHING GROUP, NEW YORK, NY, US, vol. 6, no. 1, January 2000 (2000-01), pages 107-110, XP002176627 ISSN: 1078-8956
- FERRERO: "CD14+CD34+ Peripheral Blood Mononuclear Cells Migrte Across Endothelium and Give Rise to Immunostimultory Dendritic Cells" JOURNAL OF IMMUNOLOGY, vol. 160, 1998, pages 2675-2683, XP002403428
- SANTEGOETS: "Interstitial DC and Langerhans cells, derived from the human MUTZ-3 cell line, differentiate from a common CD14+ precursor and display lymph node migration and CTL priming capabilities" IMMUNOBIOLOGY, vol. 209, 2004, page 357, XP000611720

### SEQUENCE LISTING

<110> Azienda Ospedaliera Careggi
<120> Stem cells, method for their purification and identification and their use
<130> FI 2004A000238
<150> FI 2004A000238
   <151> 2004-11-15
<160> 3
<170> PatentIn version 3.3
<210> 1
   <211> 30
   <212> DNA
   <213> Artificial
<220>
   <223> probe for human ortholog of mouse NANOG gene mapped at 12p 13.31
<400> 1
   tccatccttg caaatgtctt ctgctgagat 30
<210> 2
   <211> 23
   <212> DNA
   <213> Artificial
<220>
   <223> Reverse primer for VIC-marked probe for human ortholog of mouse NANOG gene mapped at 12p 13.31
<400> 2
   aggagagaca gtctccgtgt gag 23
<210> 3
   <211> 23
   <212> DNA
   <213> Artificial
<220>
   <223> Forward primer for VIC-marked probe for human ortholog of mouse NANOG gene mapped at 12p 13.31
<400> 3
   gatttgtggg cctgaagaaa act 23

## Description

### Field of invention

The invention relates to new stem cell populations, to their purification and use for treatment of vascular pathologies and other types of human pathologies.

### State of the art

It is by now well known that adult peripheral blood contains bone marrow-derived circulating cells capable of differentiating in mature endothelial cells (ECs), termed endothelial progenitor cells (EPCs); these cells are able to localise at the sites of ischemia contributing to formation of new blood vessels. Due to this property, these cells have been successfully used for tissue repair both in *in vivo* animal models and in patients affected by acute myocardial infarction and chronic ischemic cardiopathy.

On the other hand, it is also well known that post-ischemic infusion of terminally differentiated mature endothelial cells does not result in increased vascularization, hence it is clear that these cells lose their potency upon differentiation.

It is well known that EPCs can be obtained from CD133+ cells, from CD34+CD14- cells and from CD14+CD34- cells, even though it is still uncertain whether the latter are truly endowed with stem capacity. Furthermore, the real number of endothelial progenitors in peripheral blood and bone marrow is still a matter of discussion. CD14/CD34 double positive cells are known in prior art (Ferrero et al. 1998).

There is a clear interest for in depth studies in this field, in view of the potential importance of these cells for reconstruction of tissues damaged by ischemia and pathological or traumatic events.

It is also well known that a gene has been recently described, termed NANOG, which plays a key role in renewal and maintenance of embryonic stem cell pluripotency. NANOG expression has not yet been studied in adult stems cells.

### Description of the figures

Fig. 1 shows the differential expression of NANOG in adult cells endowed with stem capacity and in cells from other tissues.
Fig. 2 shows the expression of NANOG in cultured EPCs.
Fig. 3 shows the expression of various markers by cultured EPCs, at mRNA level.
Fig. 4 demonstrates that cultured EPCs are CD14+CD34+
Fig. 5 (A) shows the separation of CD14+CD34- cells and of CD14+CD34+ cells from circulating CD14+ cells.
Fig. 5 (B) shows the expression of NANOG in the three distinct CD14+CD34+, CD14+CD34-, CD14+ populations.
Fig. 6 shows the different ability of CD14+CD34+, CD14+CD34- and CD14-CD34- cells to differentiate in endothelial cells, and to express endothelial-specific markers.

### Detailed description of the invention

The present invention allows the use of a new population of stem cells that can be applied to tissue reconstruction therapy, and enables identification and recovery of said cells. Moreover, the disclosure allows realisation of a diagnostic kit to evaluate the percentage (and the absolute number) of circulating cells endowed with stem capacity and a kit for purification of said cells for therapeutic purposes.

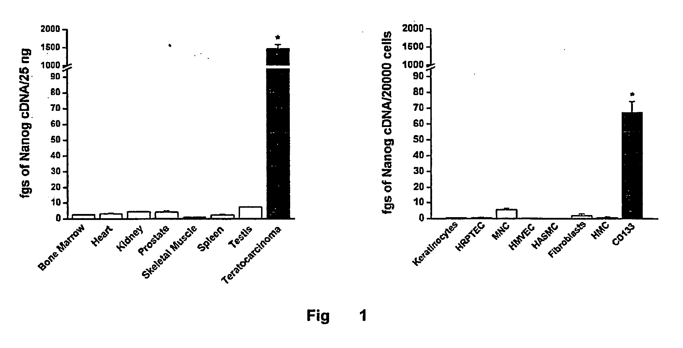

In fact, it has been surprisingly found that the NANOG gene is a useful marker for identification of adult stem cells, since it is expressed by CD133+ cells obtained from the umbilical cord or peripheral blood or from CD34+ cells. These are notoriously adult cells endowed with stem capacity. In contrast, NANOG is not expressed in primary cultures of endothelial, epithelial, smooth muscle cells, in lymphocytes or other terminally differentiated cell types (Fig. 1).

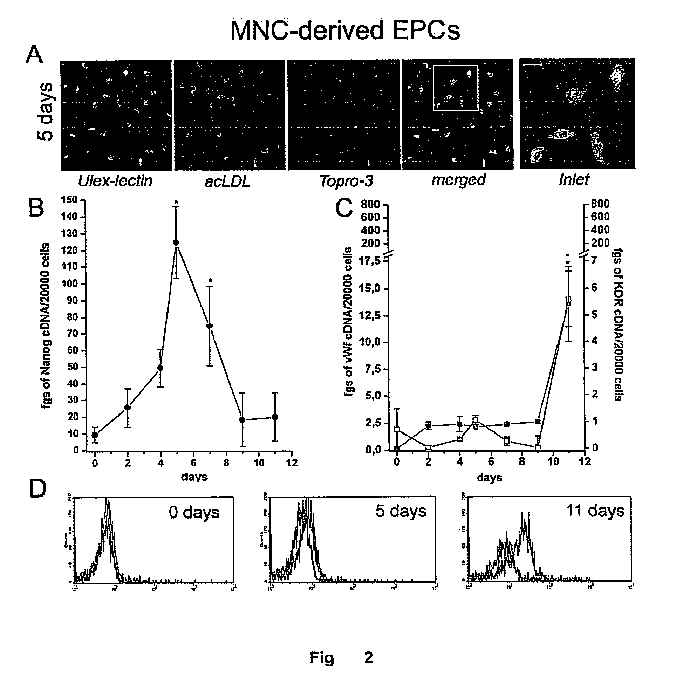

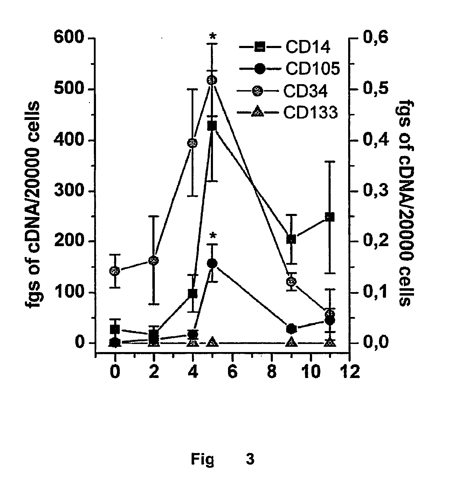

Moreover, it has been found that NANOG is expressed at high levels in mononucleated circulating cells (MNC) cultured in order to obtain EPCs, while the expression of this gene disappears when cells acquire phenotypic characteristics of terminally differentiated cells and mature endothelial cell markers, such as Von Willebrand factor, KDR and Tie-2 (Fig. 2). EPCs express high levels of NANOG, CD14+, CD34+, CD105+, as shown by Real-time quantitative RT-PCR analysis (Fig. 3).

By cytofluorimetric surface analysis, said cells are also CD14+, CD105+, CD11c+, CD31+, CD86+, HLA-DR+, whereas the presence of CD34+ on the surface of the cells of the present invention was not detectable by normal cytofluorimetric techniques (Table 1).

**TABLE 1**

| Surface markers | MNCs | EPCs |
|---|---|---|
| CD1a | 0.05 ± 0.05 | 2.3 ± 0.7 |
| CD1c | 1.75 ± 0.2 | 0.5 ± 0.1 |
| CD3 | 58.1 ± 0.1 | 7.9 ± 0.9 |
| CD11c | 32.9 ± 0.4 | 86.0 ± 1.9 |
| CD14 | 18.7 ± 1.5 | 84.9 ± 2.0 |
| CD16 | 17.4 ± 1.3 | 81.8 ± 2.8 |
| CD19 | 6.8 ± 1.0 | 2.7 ± 0.6 |
| CD31 | 60.5 ± 0.8 | 84.0 ± 4.8 |
| CD34 | 0.15 ± 0.05 | 0.7 ± 0.2 |
| CD45 | 95.4 ± 0.2 | 98.2 ± 0.4 |
| CD80 | 0.7 ± 0.2 | 3.1 ± 0.1 |
| CD86 | 19.2 ± 1.0 | 74.3 ± 6.6 |
| CD105 | 8.2 ± 1.2 | 83.1 ± 1.8 |
| CD133 | 0.2 ± 0.05 | 0.6 ± 0.3 |
| HLA-DR | 38.3 ± 0.2 | 86.5 ± 3.2 |

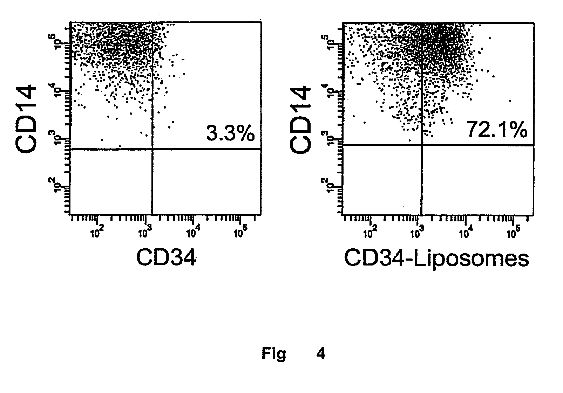

A much more sensitive method has been applied to find out whether CD34 is also expressed on the surface of said cells, as suggested by the presence of high levels of the corresponding mRNA. In particular, the cytofluorimetric technique employing antibody (Ab)-coupled magnetofluorescent liposomes (Ab-CMFL) has been used for this purpose. This technique, that can increase the fluorescent signal about 100-1000 fold, demonstrates that almost all cultured cells are CD14+CD34+ (Fig. 4).

As mentioned above, the cell population of the present invention consists of cells showing the simultaneous presence of CD14 and CD34 markers on their surface, which made possible the isolation of this population from the remaining mononucleated cells in peripheral blood. In addition to circulating blood, as mentioned above, the isolation of cells can be similarly performed from umbilical cord blood, bone marrow, placenta or adult tissues.

To verify whether the population of cultured CD14+CD34+ EPCs originated from selection of a pre-existing population in peripheral blood, mononucleated cells were isolated from the other cellular elements present in the blood, by separation in a density gradient (density = 1.077 gram/liter). CD14+ cells were purified from the population of mononucleated cells of peripheral blood by means of a positive separation method involving a magnetic column. Once the number of cells was determined, these were incubated in buffer containing anti-CD14 antibodies coupled to superparamagnetic microspheres and applied to a column in a magnetic field. The CD14+ cell fraction was recovered by elution upon removal of the column from the magnetic field.

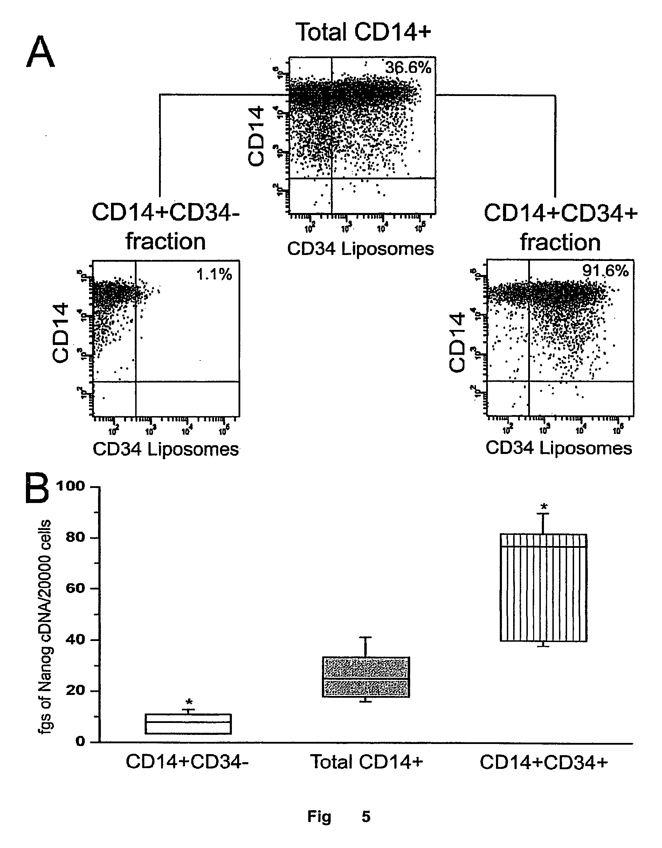

The purified CD14+ cell fraction (>95% homogeneous) was then sequentially incubated with: fluorescein coupled anti-CD34 antibodies, digoxigenin coupled anti-fluorescein antibodies, biotin coupled anti-digoxigenin antibodies and, finally, with fluorescent liposomes (Ab-CMFL) to which anti-biotin antibodies were previously coupled (Fig. 5 (A)).

After this treatment, cells were analysed by means of a sorting cytofluorimeter and the CD14+CD34+ cell population was isolated (purity > 98%). Using the Ab-CMFL technique, the percentage of CD14+ cells that also express CD34+ was measured in 10 healthy subjects with age ranging between 24 and 40 years. The percentages of CD14+CD34+ cells were comprised from 1.2 to 7.5% of all leukocytes.

To confirm that circulating CD14+CD34+ cells are the primary source of EPCs, CD14+ cells were purified from the mononucleated population and subdivided into CD34+ and CD34- cells, using the Ab-CMFL technique. The residual population of CD14-CD34- cells was also evaluated as further control. The three populations isolated by these means were then tested for NANOG expression by quantitative RT-PCR. NANOG expression turned out to be significantly higher in CD14+CD34+ cells and significantly lower in CD14+CD34- cells, as compared to non-separated CD14+ cells (Fig. 5 (B)).

CD14+CD34+, CD14+CD34- and CD14-CD34- sub-populations were cultured in presence of VEGF under the above conditions and examined on day 12 for expression of typical endothelial cell markers, such as KDR and vWf. The results have shown that, in both populations, the expression of KDR and vWf mRNAs was absent or extremely low on day 0. However, expression of these markers progressively increased in the CD14+CD34+ subgroup, reaching a maximum on day 12, while remained virtually unchanged for all the culture period in the CD14+CD34- population and in the CD14-CD34- population.

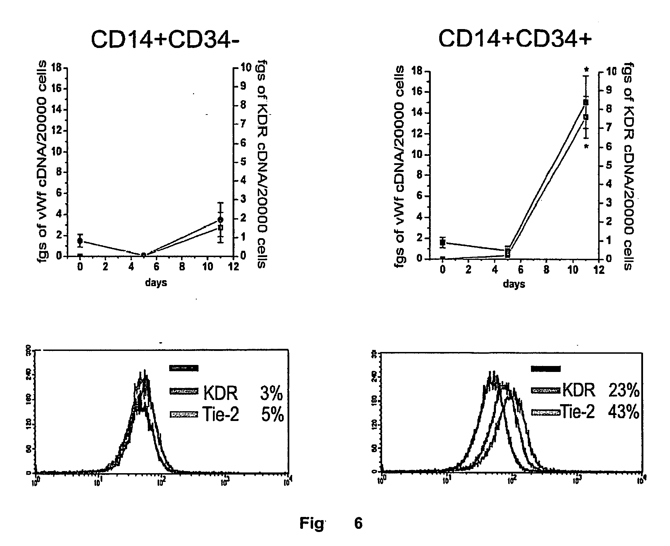

Flow cytometric tests for surface expression of KDR and Tie-2 confirmed that basically all cells of the CD14+CD34+ population expressed both KDR and Tie-2, while these same markers were virtually absent in cells derived from CD14+CD34- and CD14-CD34- cell populations (Fig. 6).

EPC cells expressing NANOG were CD14+CD34+ positive. These cells originate from selection in culture of circulating CD14+CD34+ cells expressing NANOG, which pre-exist in peripheral blood.

Hence it is evident that the CD14+CD34+ population, which is characterised by the expression of NANOG, is the main source of EPCs.

Therefore the recovery from blood and the use of CD14+CD34+ cells expressing NANOG can be important for treatment of vascular damage. Furthermore, testing for NANOG expression may represent a useful way to identify adult human cells with stem capacity. NANOG expression, a feature of pluripotent stem cells, suggests that this stem cell population may be therapeutically useful also in other types of pathologies characterised by organ or tissue damage.

Thus the invention also relates to pharmaceutical compositions that can be useful in cell therapy for treatment of ischemia, of neurodegenerative or cerebrovascular diseases, and for therapy of organ damage. (heart, kidney, pulmonary, liver or pancreatic failure and reconstruction of urogenital, visual, hearing, olfactory, cutaneous and/or mucous, bony and/or cartilaginous organs and tissues), treatment of haematological diseases and immunodeficiencies.

Moreover, the ability to determine the number of cells present in the blood represents an interesting diagnostic method to evaluate appropriateness of adopting cell therapy for treatment of the above-mentioned pathologies, based on presence or absence of a sufficient number of cells in the patient.

### EXPERIMENTAL PART

CD14+ or HMVEC cells (8x10⁶ cells/ml, cell density 2.5x10⁶ cells/cm²) were plated on human fibronectin coated culture dishes and maintained in basal endothelial medium enriched with EGM Single Quotes, VEGF (100nm/ml) and 20% FCS.

Total RNA from said cells was extracted and treated with Dnase I in order to remove any possible genomic DNA contaminant. Taq-Man RT-PCR was performed as described by Lasagni L et al. «An alternative spliced variant of CXCR3 mediates IP-10, Mig e I-TAC induced-inhibition of endothelial cell growth and acts as functional receptor for PF-4» J. Exp. Med, 2003; 197: 1537-1549.

Tie-2, KDR determination and CD1 a quantification were then performed by a Taq-Man assay using a commercial kit (Applied Biosystems, Warrington, UK).

Standard curves were generated using a series of dilutions of the mRNA prepared from HMVEC. For specific and quantitative determination of the expression of NANOG mRNA in different human tissues and cultures, we prepared different oligonucleotide pairs capable of detecting the human ortholog of the murine Nanog gene, mapped at 12p 13.31.

To ensure specificity, the entire sequence amplified by said oligonucleotides was used for tBLASTn searches of the whole human genome, at the NCBI.

The oligonucleotide pair selected on the basis of specificity was used for a quantitative Taq-man RT-PCR assay, together with a VIC-labelled fluorescent probe. Specificity and optimal amplification efficiency of said oligonucleotides were tested on a plasmid carrying the NANOG cDNA sequence.

Primers and probes are:
Nanog: VIC probe, 5'-TCCATCCTTGCAAATGTCTTCTGCTGAGAT-3': forward 5' GAT TTGTGGGCCTGAAGAAAACT-3'; REVERSE 5'-AGGAGAGACAGTCTCCGTGTGAG-3'.

mRNA levels were quantified by comparison of the experimental data with standard curves generated from a dilution series using identical amounts of plasmid DNA.

Flow cytometric analysis of cell surface molecules was performed as described in the above mentioned paper, followed by the Ab-CMFL technique (see also Scheffold A. et al. «High sensitivity immunofluorescence for detection of the pro- and anti-inflammatory cytokines gamma interferon and interleukin-10 on the surface of cytokine-secreting cells» Nat. Med. 2000; 6:107-110.).

This method makes possible to increase the intensity of the fluorescence signal 100 - 1000 fold compared to conventional methods.

Adherent cells were incubated on poly-L-lysine coated substrates for 30' at 37°C in presence of acLDL double labeled probes for one hour; after fixation, samples were incubated in presence of FITC-labeled Ulex europeus agglutinin I. Samples were mounted in anti-fading media and examined by conventional confocal microscopy.

## Claims

1. Adult human cells endowed with stem capacity and endothelial cell regeneration potential **characterised by** the simultaneous presence of CD14 and CD34 markers on their surface as well as the stem cell marker NANOG and by the fact that they express levels of CD34 detectable only by Ab-conjugated magnetofluorescent liposomes or other secondary reactants capable of detecting even low levels of CD14+ and CD34+ expressed on the cell surface.

2. Method for the identification of cells according to Claim 1 wherein the simultanoeous presence CD14, CD34 and Nanog is detected wherein the levels of CD34 are detected by Ab-conjugated magnetofluorescent liposomes or other secondary reactants capable of detecting even low levels of CD14+ and CD34+ expressed on the cell surface.

3. Use of cells according to Claim 1 for the preparation of a pharmaceutical composition for cell therapy useful for treatment of ischemia, neurodegenerative or cerebrovacsular diseases and for therapy of damage of heart, kidney, pulmonary, liver or pancreatic failure and reconstruction of urogenital, visual, hearing, olfactory, cutaneous and mucous, bony and cartilagineous organs and tissues, treatment of haematological diseases and immunodeficiencies.

## Patentansprüche

1. Humane erwachsene Zellen ausgestattet mit Stammkapazität und mit endothelialem Zellregenerationspotential **gekennzeichnet durch** die gleichzeitige Anwesenheit von CD 14- und von CD-34-Markern an ihrer Oberfläche sowie den Stammzellenmarker NANOG und **durch** die Tatsache, dass diese lediglich **durch** Ab-konjugierte magnetofluoreszente Liposomen oder andere sekundäre Reaktanden, welche selbst geringe Mengen von an der Zelloberfläche exprimierten CD 14+ und CD34+ detektieren können, detektierbare Mengen von CD34 exprimieren.

2. Verfahren zur Identifikation von Zellen nach Anspruch 1, wobei die gleichzeitige Anwesenheit von CD 14, CD34 und Nanog detektiert wird, wobei die Mengen von CD34 durch Ab-konjugierte magnetofluoreszente Liposomen oder andere sekundären Reaktanden, welche selbst geringe Mengen von auf der Zelloberfläche exprimierten CD 14+ und von CD34+ detektieren können, detektiert werden.

3. Verwendung von Zellen nach Anspruch 1 zur Herstellung einer pharmazeutischen Zusammensetzung für die Zelltherapie, welche zur Behandlung von Ischämie, neurodegenerativen oder zerebrovaskularen Erkrankungen und zur Therapie von Herzschäden, Nierenschäden, Lungenschäden, Leberschäden oder Pankreasfehlfunktion und zum Wiederaufbau von urogenitalen, optischen, Gehör-, Geruchs-, kutanen und mukösen, Knochen- und knorpeligen Organen und Geweben, zur Behandlung von hämatologischen Erkrankungen und Immundefekten geeignet ist.

## Revendications

1. Cellules humaines adultes dotées d'une capacité de cellules souches et d'un potentiel de régénérescence de cellules épithéliales, **caractérisées par** la présence simultanée des marqueurs CD14 et CD34 sur leur surface ainsi que du marqueur NANOG des cellules souches et par le fait qu'elles expriment des taux de CD34 uniquement détectables par des liposomes magnétofluorescents conjugués à un Ac ou d'autres réactifs secondaires capables de détecter des taux même bas de CD14+ et de CD34+ exprimés sur la surface cellulaire.

2. Procédé d'identification de cellules selon la revendication 1, dans lequel la présence simultanée de CD14, CD34 et Nanog est détectée, où les taux de CD34 sont détectés par des liposomes magnétofluorescents conjugués à un Ac ou d'autres réactifs secondaires capables de détecter des taux même bas de CD14+ et de CD34+ exprimés sur la surface cellulaire.

3. Utilisation de cellules selon la revendication 1 pour la préparation d'une composition pharmaceutique destinée à une thérapie cellulaire utile pour le traitement d'une ischémie, de maladies neurodégénératives ou cérébrovasculaires et pour une thérapie de lésions d'insuffisance du coeur, du rein, du poumon, du foie ou pancréatique et la reconstruction d'organes et de tissus urogénitaux, visuels, auditifs, olfactifs, cutanés et muqueux, osseux et cartilagineux, le traitement de maladies hématologiques et d'immunodéficiences.
